# EUROPÄISCHE PATENTSCHRIFT

(11) **EP 0 659 075 B1**
(45) Veröffentlichungstag und Bekanntmachung des Hinweises auf die Patenterteilung: **10.07.1996**
(21) Anmeldenummer: 93917792.9
(22) Anmeldetag: 16.08.1993
(51) Int. Cl.: A61K 9/70

(54) **VERFAHREN ZUR HERSTELLUNG EINER FLÄCHIGEN DOSIERUNGSFORM FÜR WIRKSTOFFE**
PROCESS FOR PRODUCING A FLAT ACTIVE SUBSTANCE ADMINISTRATION FORM
PROCEDE DE FABRICATION D'UNE FORME GALENIQUE PLATE POUR DOSAGE DE PRINCIPES ACTIFS

(30) Priorität: 12.09.1992 DE 4230589
(43) Veröffentlichungstag der Anmeldung: 28.06.1995
(73) Patentinhaber: LTS Lohmann Therapie-Systeme GmbH & Co. KG, D-56567 Neuwied (DE)
(72) Erfinder: MÜLLER, Walter, D-56564 Neuwied (DE); ANHÄUSER, Dieter, D-56581 Melsbach (DE)
(74) Vertreter: Flaccus, Rolf-Dieter, Dr.
(86) Internationale Anmeldenummer: EP9302168
(87) Internationale Veröffentlichungsnummer: WO9406419

(56) Entgegenhaltungen:
- EP-A- 0 215 225
- EP-A- 0 303 025
- DE-A- 1 461 280
- PATENT ABSTRACTS OF JAPAN vol. 016, no. 342 (C-0966)24. Juli 1992 & JP,A,04 100 981 (KOREHOO KK) 2. April 1992

## Beschreibung

Die Erfindung betrifft ein Verfahren entsprechend dem Oberbegriff von Anspruch 1. Dieses Verfahren soll es bei der Herstellung von im wesentlichen flächigen Arzneiformen oder Vorrichtungen gestatten, flüssige Wirkstoffe, flüssige Wirkstoffzubereitungen, und/oder flüssige Wirk-/Hilfsstoffgemische mengenmäßig genau sowie in der Fläche gleichmäßig zu den übrigen Bestandteilen der Arzneiform bzw. der Vorrichtung zu dosieren.

Solche flächigen Arzneiformen können zum Beispiel transdermale therapeutische Systeme, transmucosale Systeme, dermale, d.h. nur topisch wirkende Systeme, aber auch oral zu verabreichende Arzneiformen wie z.B. Sublingualtabletten oder Sublingualoblaten sein.
Besonders wertvoll allerdings ist das verfahren bei der Herstellung von transdermalen oder dermalen Systemen vom Matrixtyp bzw. Membransystemen mit einem nicht flüssigen Reservoir.

Da dem Fachmann die Funktion von solchen transdermalen oder dermalen therapeutischen Systemen und die zu ihrer Herstellung notwendigen Materialien hinreichend bekannt sind, sei hier nur kurz erwähnt, daß der oder die Wirkstoffe in den meistens selbstklebenden Systemen zumindest zum Teil gelöst vorliegen, und bei Applikation des Systems auf die Haut aus dem System in die Haut diffundieren und ihre Wirkung lokal oder systemisch entfalten.

Die Erfindung wird im Vergleich zum Stand der Technik nachstehend anhand von begleitenden Zeichnungen erläutert. Es zeigen:
- Fig. 1: eine bekannte Darreichungsform mit Rückschicht, selbstklebender Matrix und entfernbarer Schutzfolie;
- Fig. 2: eine Darreichungsform, umfassend Rückschicht, eine erste und zweite Matrixschicht mit dazwischen eingelagerter Substratschicht und eine entfernbare Schutzfolie, im Zustand der Laminierung;
- Fig. 3: im Schnittbild die fertig laminierten Schichten der Darreichungsform nach Fig. 2;
- Fig 4a bis 4f: eine Folge von Arbeitsschritten eines bekannten Druckvorganges in rein schematischer Darstellung;
- Fig 5a und 5b: eine Folge von Arbeitsschritten des Druckvorganges nach der Erfindung;
- Fig. 6: eine schematische Darstellung einer kontinuierlichen Herstellung von Darreichungsformen bzw. Vorrichtungen in Form eines Stammbaumes.

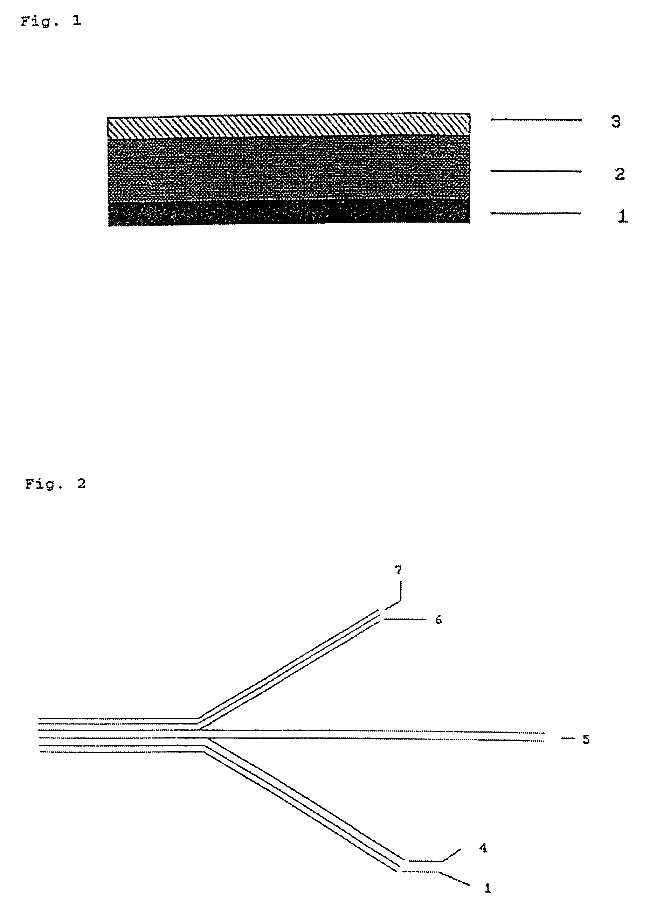

Der einfachste Fall liegt bei bekannten einschichtigen Matrixsystemen vor, die entsprechend Figur 1 aus einer undurchlässigen Rückschicht 3, einer selbstklebenden Matrixschicht 2 und einer entfernbaren Schutzschicht 1 bestehen können.

Die Matrixschichten (und dies gilt auch für Reservoirschichten der oben angesprochenen Membransysteme) werden überlicherweise in solcher Art und Weise hergestellt, daß Komponenten der Matrix sowie Wirkstoffe in einem Lösemittel gelöst auf eine geeignete Folie (entfernbare Schutzschicht 1) beschichtet und die Lösemittel in einem Trockenprozeß entfernt werden. Dieses Herstellverfahren kann zu enormen Schwierigkeiten führen, wenn Inhaltsstoffe benötigt werden, die entweder mit den Lösemitteln inkompatibel oder sehr temperaturempfindlich sind, bzw. einen bei Trocknungstemperatur zu hohen Dampfdruck aufweisen.
Beispiele für thermolabile Wirkstoffe sind z.B. Vitamin D₃-Derivate, Beispiele für Wirkstoffe mit zu hoher Flüchtigkeit sind z.B. die Wirkstoffe Nicotin oder Nitroglycerin.
Eine andere wichtige Gruppe von für dermale oder transdermale therapeutische Systeme eingesetzten Substanzen sind die sogenannten Penetrationsverstärker. Aufgabe dieser Penetrationsverstärker ist es, den Durchtritt der Wirkstoffe durch die Haut zu erleichtern.

Beispiele für Penetrationsverstärker mit hoher Flüchtigkeit sind Z.B. Terpene (Eucalyptol, Campher etc.), Ester (Ethylacetat, Ethylpropionat etc.), Alkohole (Ethanol, Propanol, Propandiol etc.) oder Ketone (Methyl-hexylketon, Methyl-octylketon etc.).

Inkompatibilitäten zwischen Lösemitteln und Inhaltsstoffen sind immer dann zu erwarten, wenn eine chemische Reaktion zwischen ihnen möglich ist. So werden z.B. für viele Klebstoffe Alkohole eingesetzt, die dann mit Wirkstoffen, die über eine freie Carboxlgruppe oder eine Estergruppe verfügen, im Sinne einer Veresterung bzw. einer Umesterung reagieren können.

Um diesen Schwierigkeiten aus dem Weg zu gehen, wurden Verfahren entwickelt, bei denen eine flüssige Zubereitung der Problemsubstanzen bei Raumtemperatur auf ein flächiges Substrat aufgebracht wird und dieses Substrat dann zwischen vorgefertigte Schichten der späteren Arzneiform gebracht wird. Die flüssige Zubereitung mit allen ihren diffusiblen Inhaltsstoffen migriert nun innerhalb von Stunden oder Tagen zur Gänze in diese Schichten. Ein solches Substrat kann z.B. aus einer Papierfolie, einem Vlies, einem Textil oder anderem saugfähigem Material bestehen. Der einfachste Fall liegt vor, wenn ein mit flüssiger Zubereitung imprägniertes Material (5) entsprechend Figur 2 zwischen ein Laminat aus einer undurchlässigen Rückschicht (3) und einer selbstklebenden Verankerungsschicht (6) und einem zweiten Laminat aus einer entfernbaren Schutzfolie (1) und einer weiteren selbstklebenden Hautkontaktschicht (4) gebracht wird.

Alternativ kann das zu imprägnierende Substrat auch vor dem Aufbringen der flüssigen Zubereitung zuerst auf eine der selbstklebenden Schichten 4 oder 6 laminiert werden.

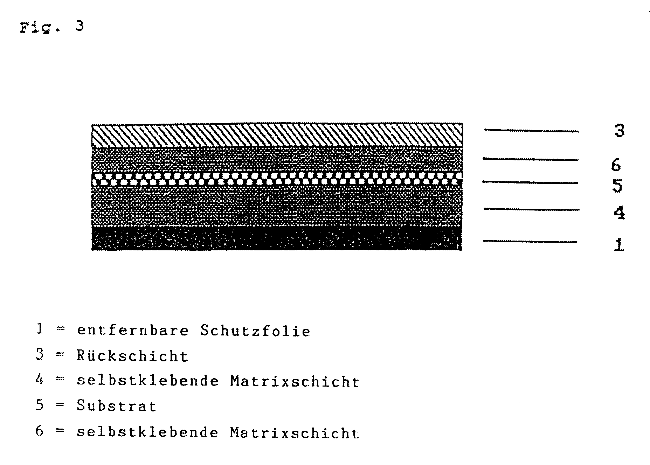

Da die fertigen Systeme (Figur 3) aus einem großflächigen Gesamtlaminat ausgestanzt werden, ist es bei der Verwendung von sehr teuren Wirk- oder Hilfsstoffen bzw. bei Wirkstoffen, die wie z.B. Narcotica ein hohes Mißbrauchspotential haben, von Vorteil, wenn die flüssige Zubereitung in Form von den späteren Systemen entsprechenden Mustern aufgebracht wird und so der entstehende Stanzabfall frei von diesen Stoffen ist.

Ein Herstellverfahren für solche Systeme ist in der US 4,915,950 beschrieben. Hier werden ganz allgemein beliebige Dosierverfahren unter dem Überbegriff Druckverfahren zusammengefaßt. Speziell erwähnt werden dabei Tiefdruck, Extrusionsbeschichtung, Siebdruck, Sprayen und Streichen.

In keinem der angeführten Beispiele wird jedoch genau beschrieben, welches spezielle Druckverfahren zu seiner Herstellung benutzt wurde.
Es kann deshalb auch nicht beurteilt werden, inwieweit das oder die zu ihrer Herstellung angewandten Verfahren den an Arzneimitteln zu stellenden Anforderungen hinsichtlich Genauigkeit der Dosierung gerecht werden.

Ein weiteres Beispiel für ein System, bei dem auf einem Trägermaterial wirkstoffhaltige Zonen und wirkstoffreie Abfall-Zonen aufgebracht und anschließend durch einen Stanzvorgang getrennt werden, ist in der DE 35 31 795 A1 beschrieben.
Dabei soll eine genaue Dosiermöglichkeit der Wirkstoffe mittels exakt gravierter bzw. geätzter Druckwalzen oder Druckplatten erreicht werden, wobei die Auftrennung in wirkstofffreie und wirkstoffhaltige Zonen mittels nicht näher beschriebener drucktechnischer Verfahren - genannt werden Siebdruck-, Flexodruck-, Tiefdruck- oder berührungslose Druckverfahren wie Ink-Jetting bzw. Aufspritzen durch Düsen etc. - erfolgt. Es fehlen jedoch Angaben darüber, wie bei den bekannten Druckverfahren die Einhaltung vorgegebener exakter Dosiermengen von Wirkstoffen mit definierten Flächenkonzentrationen erreicht werden soll. Dies ist offenbar auch nicht wichtig, weil es sich um Mottenschutzpapier handelt, welches einen Insektizid-Duftstoff an die Umgebungsluft über längere Zeiträume abgibt, und im Gegensatz zu einer Darreichungsform mit Hautkontakt keine exakte Flächenkonzentration aufweisen muß.

In der OS 37 27 232 wird ein spezielles Druckverfahren, ein sogenanntes Tamponprintverfahren, zur Wirkstoffdosierung bei der Herstellung von transdermalen oder dermalen Systemen beschrieben.

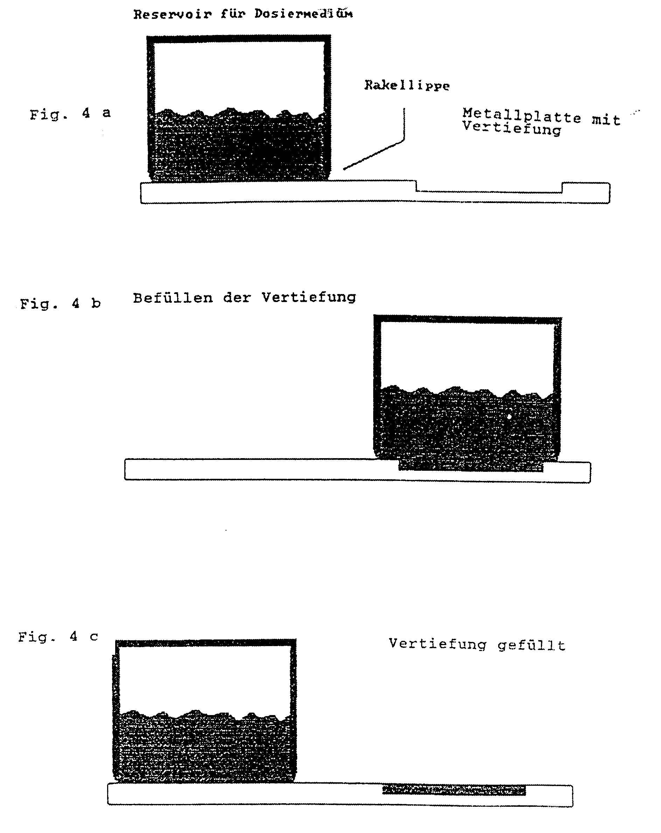

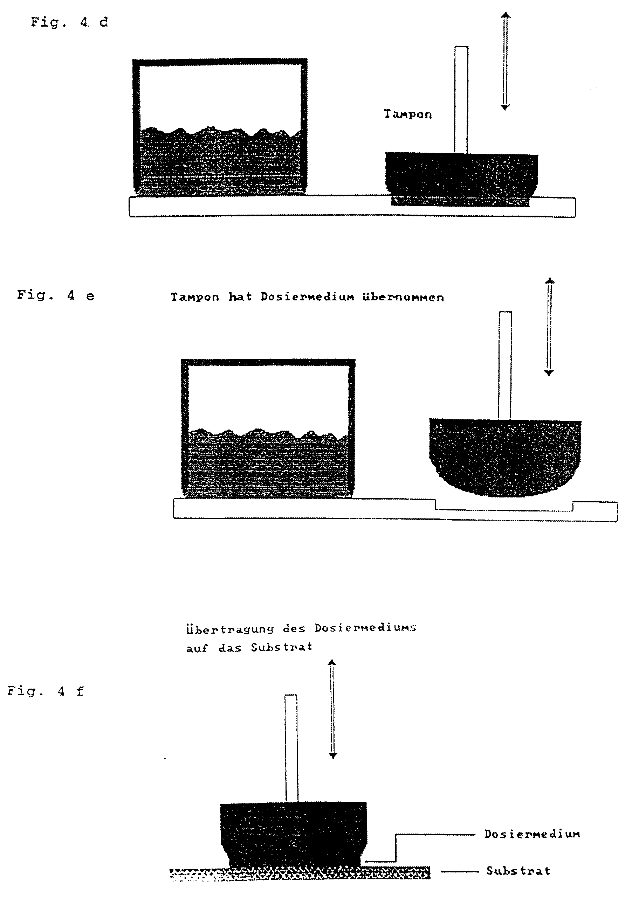

Tampondruckverfahren in ihrer modernen Form sind seit 1968 bekannt und, ein Tampondruckwerk ist beispielsweise in der DE-OS 19 39 437 beschrieben. Diese Drucktechnik ist speziell zum Bedrucken von unebenen Oberflächen geeignet, da sich ein verformbares, das Druckmedium übertragendes Tampon den zu bedruckenden Substraten anpaßt.
Das zu druckende Bildelement ist bei diesem Verfahren in eine Metallplatte eingeätzt. Das Druckmedium - im folgenden Text jeweils Dosiermedium genannt - wird in diese geätzte Vertiefung überführt (Figur 4a), mittels eines Rakelvorgangs dosiert (Figur 4b u. c), anschließend von dem Tampon übernommen (Figur 4d u. e) und auf den zu bedruckenden Gegenstand übertragen ( Figur 4f ).

Nachteilig bei diesem Verfahren ist, daß die übertragenen Wirkstoffmengen von einer ganzen Reihe von Faktoren abhängen. In erster Linie werden sie bestimmt durch die Ätztiefe der Druckform, aber auch z.B. durch die Viskosität und Kohäsion des Dosiermediums, der Adhäsion des Dosiermediums zu dem Plattenmaterial und der Härte und Oberflächenbeschaffenheit der eingesetzten Tampons. Es ist deshalb u.U. schwierig, diese Faktoren so aufeinander abzustimmen, daß das gewünschte Dosiergewicht erreicht und über längere Fertigungszeiten eingehalten wird. Speziell bei größeren Flächen und bei von einer kreisförmigen Geometrie abweichenden Druckbildern ist es sehr schwierig, eine gleichmäßige Verteilung des Dosiermediums in der Fläche zu erreichen. Die gleichmäßige Verteilung des Dosiermediums in der Fläche ist jedoch bei der Herstellung von transdermalen oder dermalen therapeutischen Systemen von besonderer, ausschlaggebender Bedeutung.

So bewirkt eine ungleichmäßige Verteilung des Wirkstoffs in der Fläche im Falle des dermalen Systems eine nicht am ganzen Applikationsort gleich intensive Wirkung, und im Falle eines transdermalen Systems kann die systemisch verfügbare Wirkstoffmenge auch von der Wirkstoffverteilung beeinflußt werden.

Oft beeinflußt die Konzentration des flüssigen oder halbfesten Dosiermediums in der Arzneiform auch deren physikalische Eigenschaften. Bei dermalen oder transdermalen Systemen, die in den allermeisten Fällen auf ihrer gesamten Kontaktfläche zur Haut selbstklebend sind, ist davon vor allem die Klebkraft und Kohäsion betroffen. So können Areale mit zu hoher Konzentration zu weich und dadurch zu aggressiv klebend werden, während Areale mit zu niedriger Konzentration möglicherweise zu schwach kleben, und deshalb der für die Funktion der Systeme wichtige intensive Kontakt zur Haut nicht mehr gewährleistet ist.

Es war deshalb Aufgabe dieser Erfindung, ein neues Verfahren zum präzisen flächigen Dosieren von flüssigen Zubereitungen speziell für die Herstellung von einzeldosierten Arzneiformen, insbesondere für die Herstellung von einzeldosierten Arzneiformen, insbesondere für die Herstellung von transdermalen und dermalen Systemen, zu entwickeln, das die Nachteile der oben beschriebenen Verfahren vermeidet.

Die Aufgabe wird erfindungsgemäß durch ein Verfahren entsprechend den im Kennzeichnungsteil von Anspruch 1 genannten Verfahrensschritten gelöst.

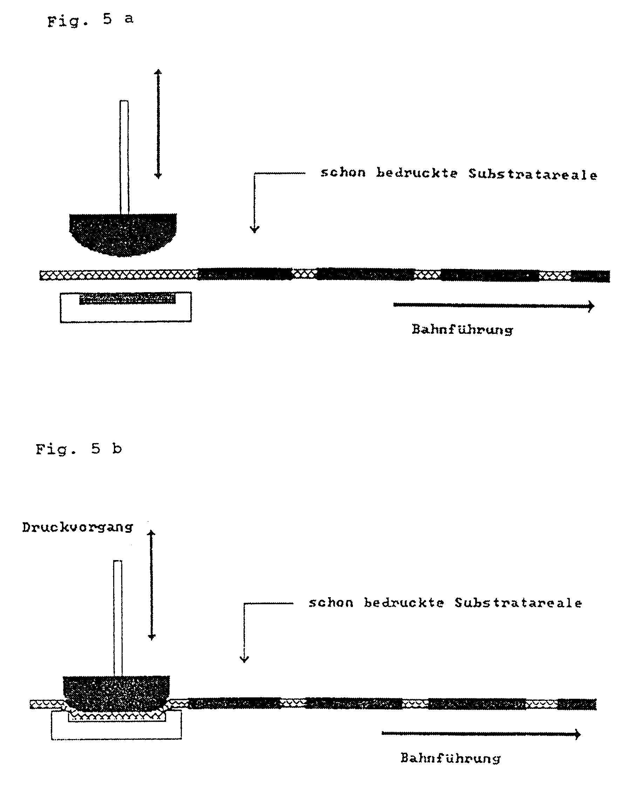

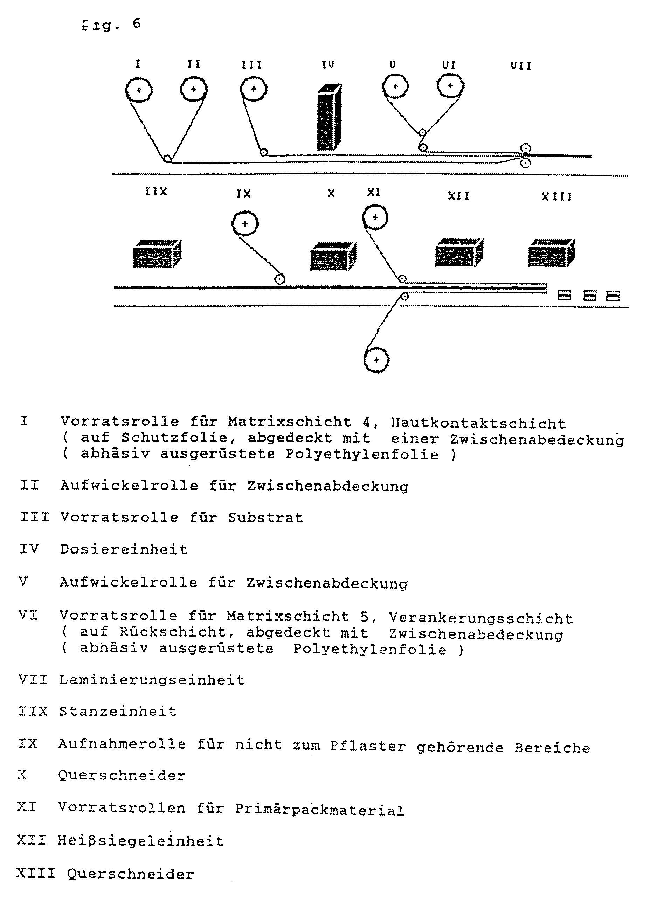

Das Prinzip der Erfindung ist in den Figuren 5a - 5b sowie in Fig. 6 verdeutlicht.

Zunächst wird wie in Figur 4a - 4c die Vertiefung mit dem Dosiermedium befüllt und anschließend abgerakelt.
Im Unterschied zum beschriebenen Stand der Technik wird dann allerdings das zu bedruckende Substrat als bahnförmiges Material über die Vertiefung geführt (Figur 5 a).
Dies ist möglich, wenn das Reservoir für das Dosiermedium in seiner Position fixiert und die Platte mit der Vertiefung der sich bewegende Teil beim Füllvorgang ist.
Über eine mechanische Vorrichtung wird nun das bahnförmige Substratband in die gefüllte Vertiefung gedrückt und nimmt das Dosiermedium auf (Figur 5 b). Die Bahnspannung sorgt dann dafür, daß sich das Band wieder aus der Vertiefung löst.
Für die mechanische Vorrichtung, die das Material in die befüllte Vertiefung drückt, sind eine Vielzahl von Möglichkeiten denkbar.
So kann sie z.B ein weiches Tampon sein, das eine Auf- und Abbewegung durchführt. Eine andere Möglichkeit besteht darin, eine dehnbare Membran - etwa wie bei einer Trommel - als Abschluß auf einen Hohlkörper zu setzen, und diesen Hohlkörper im Produktionstakt mit Überdruck zu beaufschlagen. Die sich wölbende Membran drückt dabei das Substrat in die Vertiefung.

Da es sich bei diesem Verfahren im Grunde um eine vom Prinzip her sehr genaue Volumendosierung handelt, und der Transfer mittels des Tampons entfällt, erhält man gegenüber bekannten Verfahren, z.B. nach der DE-OS 37 27 232, einen großen Zugewinn an Genauigkeit und Zuverlässigkeit. Da das Substrat im Gegensatz zu einem Tampon über aufsaugende Eigenschaften verfügen kann, ist auch die maximal zu übertragende Menge an Dosiermedium wesentlich größer.
Das Zudosieren von problematischen Inhaltsstoffe im Sinne der Erfindung ist einfach, wenn diese Inhaltsstoffen selbst bei Raumtemperaturen flüssig sind. In anderen Fällen gelingt es oft, Lösemittel zu finden, die in dem fertigen System ohne Schaden für den Anwender verbleiben können, oder es ist möglich, das Dosiermedium zu schmelzen.

Da gerade die Penetrationsbeschleuniger in vielen Fällen Flüssigkeiten darstellen, ist es oft möglich, den Wirkstoff in diesen Penetrationsbeschleunigern zu lösen und zusammen mit diesen zu dosieren.

Für das Verfahren müssen die Dosiermedien eine gewisse Mindestviskosität aufweisen. Dabei können viskositätserhöhende Zusätze wie z.B. Aerosile oder Polymere hilfreich sein, die entweder natürlichen Ursprungs wie z.B. Gelatine, Stärkederivate oder synthetischen Ursprungs wie z.B. Polyacrylsäurederivate sein können.
Ein System des in Figur 3 gezeigten beispielhaften Aufbaus wird im Sinne der Erfindung analog des in Figur 6 gezeigten Produktionsschemas sowie entsprechend dem Verfahrensanspruch 14 hergestellt.

In Position I des Schemas befindet sich eine Vorratsrolle der sich auf der Schutzfolie befindlichen selbstklebenden Matrixschicht 4, die nach Applikation Kontakt mit der Haut hat und mit einer wieder entfernbaren Folie abgedeckt ist.
Diese Folie wird entfernt und in Position II auf eine Rolle gewickelt. In Position III befindet sich eine Vorratsrolle für das Substrat 5.
Position IV stellt die Dosierstation für die flüssige Zubereitung dar. In Position VI ist eine Vorratsrolle für die selbstklebende Matrixschicht 6, die sich zwischen der Rückschicht und einer wiederentfernbaren Folie befindet.
Die wiederentfernbare Folie wird in Position V von einer Aufwicklung aufgenommen.
In Position VII wird das bedruckte Substrat zwischen die beiden Matrixschichten laminiert (siehe auch Figur 2).
Durch sich anschließende Stanzvorgänge werden die einzelnen Pflaster bzw. transdermale oder dermale Systeme gewonnen.

Die zudosierten Inhaltsstoffe diffundieren nun in Übereinstimmung mit den Fick'schen Diffusionsgesetzen in die Matrixschichten. Dieser Vorgang ist normalerweise schon nach wenigen Tagen abgeschlossen.

In einer Abwandlung des Verfahrens wird schon vor der Dosierstation die Rückschicht mit Matrixschicht 6 mit dem Substrat 5 laminiert und dementsprechend in Position VII nur noch die sich auf der Schutzfolie befindliche Matrixschicht 4 zukaschiert.

Andere Abwandlungen des Verfahrens sind denkbar und liegen im Rahmen dieser Erfindung.

Das Verfahren führt zu einer sehr gleichmäßigen Verteilung der zudosierten Inhaltsstoffe in den Systemen. Die erreichbare Genauigkeit und Reproduzierbarkeit sind so gut, daß die nach diesem Verfahren hergestellten Arzneiformen die gemäß den Pharmacopöen an Arzneimittel zu stellenden Forderungen erfüllen.

Dem erfindungsgemäßen Verfahren sind dabei Pflaster zugänglich, deren Maximalausdehnung in einer Richtung bis zu 15 cm betragen kann. Ein quadratisches Pflaster kann deshalb bis zu 225 cm² groß sein.

Bei derart großen Formaten wird dann allerdings vorteilhaft die zu befüllende Vertiefung in kleinere Einzelareale aufgeteilt, die durch schmale Stege voneinander getrennt sind.
Diese Stege können, wenn erforderlich sehr schmal, d.h. ca. 0,2 mm breit, ausgeführt werden und ihr Anteil an der Gesamtfläche ist damit sehr klein.

Handelt es sich bei den zudosierten Inhaltsstoffen um sehr flüchtige Substanzen, werden die einzelnen Pflaster mit Vorteil on-line direkt verpackt. Als bevorzugtes Verpackungsmaterial dienen Vierrandsiegelbeutel. Als nahezu immer geeignet erwiesen sich dabei Vierrandsiegelbeutel, deren innerste Schicht aus Polymeren auf Basis von Acrylnitril besteht, als besonders geeignet soll hier das Polymerisat Barex® (BP Chemicals) genannt werden.

Bei Verwendung einer on-line Verpackung ist es auch möglich, problematische Inhaltsstoffe im Sinne der Erfindung in einschichtige Matrixsysteme einzuarbeiten. Voraussetzung ist allerdings, daß die Rückschicht des fertigen Pflasters für das Dosiermedium durchlässig ist und die zu dosierenden Substanzen eine gewisse Flüchtigkeit besitzen.

Materialien für eine durchlässige Rückschicht sind z.B. textile Materialien, Folien aus Polyurethan oder Ethylen-Vinylacetat-Copolymeren. Das Dosiermedium wird bei solchen Systemen auf die Rückschicht bzw. das Trägergewebe des schon ausgestanzten Pflasters dosiert und dann die Pflaster sofort im Primärpackmittel verpackt.
Eine andere Möglichkeit zur Herstellung solcher einschichtigen Systeme ist es, die problematischen Inhaltsstoffe auf ein gesondertes saugfähiges flächiges Material zu dosieren und dieses zusammen mit dem Pflaster, idealerweise in Kontakt mit dessen durchlässiger Rückschicht, zu verpacken.
In dem undurchlässigen Primärpackstoff (Vierrandsiegelbeutel) diffundiert sodann das Dosiermedium in die selbstklebende Matrix des Pflasters.

Systeme mit einer durchlässigen Rückschicht geben nach dem Entfernen aus dem Primärpackmittel ihre flüchtigen Inhaltsstoffe auch an die Umgebungsluft ab. Während dies für eine Arzneiform nachteilig sein kann, kann man diesen Effekt mit Vorteil für die Herstellung von Vorrichtungen nutzen, die Duftstoffe, Insektizide oder Insektenreppelents abgeben.
Wenn eine solche Vorrichtung auf die Haut geklebt werden soll, kann man der Resorption dieser Mittel durch die Haut vorbeugen, indem man in die Vorrichtung eine undurchlässige Folie integriert.

Der Herstellvorgang solcher Vorrichtungen ist ansonsten der oben detailliert beschriebenen Herstellung von dermalen oder transdermalen Systemen - zumindest was die im Sinne dieser Erfindung relevanten Fertigungsschritte angeht - identisch.

### Beispiel:

Herstellung eines transdermalen therapeutischen Systems mit Eucalyptol als Penetrationsbeschleuniger gemäß Aufbau Fig. 3 und Produktionsschema Fig. 6.

Die selbstklebende Matrixschicht 1 hat ein Flächengewicht von 25 g/m² und ist mit einer abhäsiv ausgerüsteten ( silikonisierten) Polyethylenfolie als Zwischenabdeckung geschützt.
Die Rückschicht besteht aus einer 12 µm dicken Polyesterfolie.

Die ebenfalls selbstklebende Matrixschicht 2 hat ein Flächengewicht von 100 g/ m² und befindet sich auf einer 100 µm dicken, abhäsiv ausgerüsteten Polyesterfolie. Sie ist ebenfalls mit einer abhäsiv ausgerüsteten Polyethylenfolie als Zwischenabdeckung geschützt.

Beide Matrixschichten enthalten schon den Wirkstoff, sodaß lediglich der Penetrationsbeschleuniger Eucalyptol zudosiert werden muß.
Eucalyptol ist eine sehr flüchtige und dünnflüssige Substanz. Durch den Zusatz von 3 % Ethylcellulose wird die Viskosität auf ca. 3 Pas·s gesteigert und kann so im Sinne der Erfindung eingesetzt werden.

Das zu bedruckende Substrat besteht aus einem Langfaserpapier und hat ein Flächengewicht von 30 g/m².

Das Pflaster hat eine Größe von 32 cm² (4 x 8 cm) und ist rechteckig mit abgerundeten Ecken.

Die Vertiefung in der Dosierplatte hat eine Tiefe von 140 µm und ist in seinen Dimensionen jeweils 1 mm größer als das Pflaster.

Wie im Text beschrieben, wird von beiden Matrixschichten die Schutzfolie aus Polyethylen entfernt, das Langfaserpapier bedruckt und zwischen beide Matrixschichten laminiert.
Anschließend wird das einzelne Pflaster aus dem bandförmigen Laminat gestanzt und on-line verpackt.

Die zudosierte Menge an Eucalyptol betrug 140 µg/Pflaster oder 4,3 mg/ cm² und die Standardabweichung ( n = 10 ) lag bei 2,6 %.

## Patentansprüche

1. Verfahren zur Herstellung einer flächigen Darreichungsform mit einem dosierbaren Anteil an Arzneistoffen oder einer flächigen Vorrichtung zur dosierbaren Abgabe von flüchtigen Stoffen wie Duftstoffen an die Umgebungsluft unter Verwendung leicht flüchtiger oder thermolabiler Dosiermedien in flüssigem oder halbfestem Zustand als Inhaltsstoffe der Darreichungsform oder der Vorrichtung mittels drucktechnischer Verfahren, wobei eine fließfähige Zubereitung der Inhaltsstoffe in wenigstens eine volumetrisch definierte Vertiefung einer flachen Klischeeplatte in dosierter Menge hineingerakelt wird, dadurch gekennzeichnet, daß ein mit den Inhaltsstoffen bzw. Dosiermedien zu befrachtendes Substrat als bahnförmiges Material unter Bahnspannung schrittweise über die Vertiefung der Klischeeplatte geführt und mittels einer mechanischen Druckvorrichtung in die befüllte Vertiefung gedrückt wird, wobei das Substrat den Inhaltsstoff aus der Klischeeplatte in dosierter Menge aufnimmt.

2. Verfahren nach Anspruch 1, dadurch gekennzeichnet, daß das mit Inhaltsstoffen bedruckte Substrat als Bestandteil der Darreichungsform dieser hinzugefügt, beispielsweise auflaminiert wird.

3. Verfahren nach Anspruch 1, dadurch gekennzeichnet, daß das mit Inhaltsstoffen bedruckte Substrat mit der Darreichungsform so in Kontakt gebracht wird, daß es die Inhaltsstoffe durch Diffusion an die Darreichungsform bzw. an die Vorrichtung abgibt.

4. Verfahren nach Anspruch 1, dadurch gekennzeichnet, daß die Darreichungsform ein dermal zu applizierendes System ist.

5. Verfahren nach Anspruch 1, dadurch gekennzeichnet, daß die Darreichungsform ein oral applizierbares System ist.

6. Verfahren nach Anspruch 2, dadurch gekennzeichnet, daß das dermal applizierbare System ein transdermales therapeutisches System ist.

7. Verfahren nach Anspruch 2, dadurch gekennzeichnet, daß das dermal applizierbare System ein topisch wirkendes therapeutisches System ist.

8. Verfahren nach einem der vorangehenden Ansprüche, dadurch gekennzeichnet, daß das Dosiermedium wirkstoffhaltig ist.

9. Verfahren nach einem der vorangehenden Ansprüche, dadurch gekennzeichnet, daß das Dosiermedium wirkstofffrei ist und lediglich Hilfsstoffe enthält.

10. Verfahren nach einem der vorangehenden Ansprüche, dadurch gekennzeichnet, daß das Dosiermedium einen sogenannten Penetrationsbeschleuniger enthält.

11. Verfahren nach einem der vorangehenden Ansprüche, dadurch gekennzeichnet, daß der/die Wirkstoff(e) in einem oder einer Mischung von mehreren Penetrationsbeschleunigern gelöst oder suspendiert sind.

12. Verfahren nach Anspruch 1, dadurch gekennzeichnet, daß das Dosiermedium einen Duftstoff enthält.

13. Verfahren nach Anspruch 1, dadurch gekennzeichnet, daß das Dosiermedium ein Insektizid oder ein Insekten Repellent enthält.

14. Verfahren zur kontinuierlichen, insbesondere vollautomatischen Herstellung der Darreichungsform bzw. Vorrichtung nach einem oder mehreren der Ansprüche 1 bis 13, gekennzeichnet durch die Arbeitsschritte:
I- von einer auf eine Schutzfolie (1) aufgebrachten selbstklebenden ersten Matrixschicht (4), die nach Applikation der Darreichungsform Kontakt mit der Haut hat, wird eine abhäsiv ausgerüstete Zwischenabdeckung abgezogen
II- und die Zwischenabdeckung auf eine Rolle gewickelt;
III- Von einer Vorratsrolle wird Substrat (5) in Bahnform abgewickelt und schrittweise unter Bahnspannung über eine Dosierstation geführt und
IV- darin taktweise in Abständen auf die mit Dosiermedium gefüllte Vertiefung der Klischeeplatte gedrückt und mit Inhaltsstoffen in dosierbarer Menge bedruckt;
V- von einer auf eine abhäsiv ausgerüstete Schutzfolie aufgebrachten selbstklebenden zweiten Matrixschicht (6) wird die Schutzfolie abgezogen und
VI- auf eine Rolle gewickelt;
VII- das bedruckte Substrat (5) wird zwischen die beiden Matrixschichten (4) bzw. (6) laminiert
VIII- durch einen nachfolgenden Stanzvorgang wird die wirkstoffhaltige Darreichungsform von umgebenden wirkstofffreien Abfallteilen getrennt und
IX- der wirkstofffreie Abfall auf eine Abfallrolle aufgewickelt
X- mit einem Querschneider werden die Darreichungsformen bzw. Vorrichtung vereinzelt und
XI- in einer Verpackungsstation zunächst von Verpackungsmaterialstreifen abgedeckt, sodann
XII- durch Heißsiegelung ringsum versiegelt, und schließlich
XIII- durch Querschneiden in einzelne dicht versiegelte Einheiten getrennt.

## Claims

1. A process for the production of a flat administration form having a drug portion capable of being dosed or of a flat-shaped device for the dosable release of volatile substances, such as aromatic substances, to the ambient air by using high-volatile or thermolabile dosing media in liquid or semisolid state as the ingredients of the administration form or of the device by means of printing processes, wherein a flowable preparation of the ingredients is knife-coated in a measured amount into at least one volumetrically defined sink of a flat printing block, characterized in that a substrate to be charged with the ingredients or dosing media, respectively, is gradually led as a web-shaped material under web tension over the cavity of the printing block and pressed into the filled cavity by means of a mechanical pressure device whereby the substrate takes up the ingredient from the printing block in measured amount.

2. The process according to claim 1 characterized in that the substrate printed with ingredients is added to the administration form as a component thereof, for example is laminated thereon.

3. The process according to claim 1 characterized in that the substrate printed with ingredients is brought into contact with the administration form in such a manner that it releases the ingredients to the administration form or to the device by means of diffusion.

4. The process according to claim 1 characterized in that the administration form is a dermally applicable system.

5. The process according to claim 1 characterized in that the administration form is an orally applicable system.

6. The process according to claim 2 characterized in that the dermally applicable system is a transdermal therapeutic system.

7. The process according to claim 2 characterized in that the dermally applicable system is a topically effective therapeutic system.

8. The process according to any one of the preceding claims characterized in that the dosing medium comprises active substances.

9. The process according to any one of the preceding claims characterized in that the dosing medium is active substance-free and merely comprises adjuvants.

10. The process according to any one of the preceding claims characterized in that the dosing medium comprises a so-called penetration enhancer.

11. The process according to any one of the preceding claims characterized in that the active substance(s) is/are dissolved or suspended in one or in a mixture of several penetration enhancers.

12. The process according to claim 1 characterized in that the dosing medium comprises an aromatic substance.

13. The process according to claim 1 characterized in that the dosing medium comprises an insecticide or an insect repellent.

14. A process for the continuous, in particular fully automatic production of the administration form or device according to one or several of claims 1 to 13, characterized by the steps:
i- an abhesive intermediate cover is removed from a self-adhesive first matrix layer (4) which is positioned on a protective film (1) and is in contact with the skin after application of the administration form,
ii- and the intermediate cover is wound on a roll;
iii- substrate (5) is unwound from a supply roll in the form of a web and gradually led under web tension over a dosing station and
iv- in said dosing station is pressed cyclically and with distances onto the cavity of the printing block, which is filled with dosing medium, and is printed with ingredients in a dosable amount;
v- the protective film is removed from a self-adhesive second matrix layer (6) which is placed on an abhesive protective film and
vi- is wound on a roll;
vii- the printed substrate (5) is laminated between the two matrix layers (4) and (6),
viii- the active substance-containing administration form is separated from surrounding active substance-free waste portions by means of a subsequent punching procedure, and
ix- the active substance-free waste is wound on a waste roll,
x- the administration forms or devices are separated by means of a cross cutter and
xi- in a packing station are covered by strips of packing material first, then
xii- sealed on all sides by means of hot sealing, and finally
xiii- separated into individual, tightly sealed units by means of cross-cutting.

## Revendications

1. Procédé pour la réalisation d'une forme d'administration plate, présentant une teneur dosable en médicaments, ou d'un dispositif plat pour la libération dosable de produits liquides tels que des parfums dans l'air environnant, par recours à un fluide dosé, facilement volatile ou thermolabile, à l'état liquide ou semi-solide, comme substance contenue dans la forme d'administration ou dans le dispositif, au moyen d'un procédé technique d'impression, dans lequel une préparation des substances contenues, présentant une aptitude à l'écoulement, est raclée en quantité dosée dans au moins un creux volumétriquement défini d'une plaque plate de cliché, caractérisé en ce qu'un support, présentant la forme d'une bande, à charger en les substances contenues ou les fluides dosés, est guidé pas à pas à l'état tendu au-dessus du creux de la plaque de cliché, et est poussé dans le creux rempli, au moyen d'un dispositif mécanique de pression, le support recevant la substance contenue de la plaque de cliché, en quantité dosée.

2. Procédé selon la revendication 1, caractérisé en ce que le support imprimé avec les substances contenues, et servant de composant de la forme d'administration, est ajouté à ce dernier, par exemple par lamification.

3. Procédé selon la revendication 1, caractérisé en ce que le support imprimé avec les substances contenues est amené en contact avec la forme d'administration, de telle sorte qu'il émet par diffusion les substances contenues sur la forme d'administration ou sur le dispositif.

4. Procédé selon la revendication 1, caractérisé en ce que la forme d'administration est un système à appliquer sur la peau.

5. Procédé selon la revendication 1, caractérisé en ce que la forme d'administration est un système pouvant être appliqué oralement.

6. Procédé selon la revendication 2, caractérisé en ce que le système susceptible d'une application sur la peau est un système thérapeutique transdermique.

7. Procédé selon la revendication 2, caractérisé en ce que le système à appliquer sur la peau est un système thérapeutique à action topique.

8. Procédé selon l'une des revendications précédentes, caractérisé en ce que le fluide dosé contient un principe actif.

9. Procédé selon l'une des revendications précédentes, caractérisé en ce que le fluide dosé est exempt de principe actif et contient uniquement des matières auxiliaires.

10. Procédé selon l'une des revendications précédentes, caractérisé en ce que le fluide dosé contient ce que l'on appelle un accélérateur de pénétration.

11. Procédé selon l'une des revendications précédentes, caractérisé en ce que le ou les principes actifs sont dissous ou mis en suspension dans un accélérateur de pénétration ou dans un mélange de plusieurs accélérateurs de pénétration.

12. Procédé selon la revendication 1, caractérisé en ce que le fluide dosé contient un parfum.

13. Procédé selon la revendication 1, caractérisé en ce que le fluide dosé contient un insecticide ou un produit repoussant les insectes.

14. Procédé en vue de la fabrication en continu, et en particulier la fabrication entièrement automatisée, de la forme d'administration ou du dispositif selon l'une ou plusieurs des revendications 1 à 13, caractérisé par les étapes de travail suivantes :
I- un recouvrement intermédiaire non adhérent est détaché d'une première couche matricée (4) autocollante appliquée sur une feuille de protection (1), et qui après application de la forme d'administration est en contact avec la peau,
II- et le recouvrement intermédiaire est enroulé sur un rouleau;
III- le support (5) en forme de bande est déroulé d'un rouleau de réserve et est amené pas à pas, sous tension de la bande, au-dessus d'un poste de dosage, et
IV- y est, pas à pas, à intervalles, poussé contre le creux de la plaque de cliché rempli de fluide à doser, et est imprimé en quantité dosée avec les substances contenues;
V- la feuille de protection est enlevée d'une seconde couche matricée autocollante (6) appliquée sur une feuille de protection non adhérente, et
VI- est enroulée sur un rouleau;
VII- le support imprimé (5) est lamifié entre les deux couches matricées (4) et (6),
VIII- la forme d'administration contenant le produit actif est alors séparée, dans une opération d'estampage, des parties environnantes de chute exempte de principe actif, et
IX- la chute exempte de produit actif est enroulée sur un rouleau de chute,
X- les formes d'administration ou le dispositif sont découpés individuellement par un couteau transversal, et
XI- sont tout d'abord recouverts de bandes de matériau d'emballage, dans un poste d'emballage, et ensuite
XII- scellés à la chaleur sur un anneau périphérique, et enfin,
XIII- sont séparés par découpe transversale en unités individuelles scellées de manière étanche.
